# EUROPEAN PATENT APPLICATION

(11) **EP 2 878 301 A1**
(43) Date of publication of application: **03.06.2015**
(21) Application number: 13822662.6
(22) Date of filing: 25.02.2013
(51) Int. Cl.: A61K 31/4422, A61K 31/22, A61K 9/28, A61P 9/12

(54) **COMPOUND PREPARATION OF LERCANIDIPINE AND ATORVASTATIN**

(30) Priority: 24.07.2012 CN 201210258175
(71) Applicant: Zhaoke Pharmaceutical (Guangzhou) Company Limited, Guangdong 511400 (CN)
(72) Inventor: LI, Xiaoyi, Hong Kong (CN); ZHANG, Guohui, Hefei Anhui 230088 (CN); DAI, Xiangrong, Hefei Anhui 230088 (CN); LING, Juan, Hefei Anhui 230088 (CN); HU, Daidi, Hefei Anhui 230088 (CN)
(74) Representative: Von Kreisler Selting Werner - Partnerschaft von Patentanwälten und Rechtsanwälten mbB
(86) International application number: PCT/CN2013/071836
(87) International publication number: WO 2014/015672

(57) **Abstract**

The present invention relates to the medical field, discloses a compound preparation of lercanidipine hydrochloride and atorvastatin calcium, which has a drug content per unit dosage of 5-20 mg of lercanidipine hydrochloride and 10-80 mg of atorvastatin calcium. The present invention also particularly provides a compound preparation in the form of tablets, which comprises lercanidipine hydrochloride and atorvastatin calcium as main drugs, and calcium carbonate, microcrystalline cellulose, sodium carboxymethyl cellulose, hydroxypropyl cellulose, polysorbate 80, magnesium stearate, pregelatinized starch and colloidal silicon dioxide as auxiliary materials, and opadry as a coating. A clinical test shows that the compound preparation of lercanidipine and atorvastatin calcium according to the present invention is effective in significantly reducing hypertension and lipids; the medical dose is reduced, the incidence of adverse effects is lowered, and the tolerance and drug therapy compliance of patients is good, thus the compound preparation has a good clinical application prospect.

## Description

The application claims the priority of Chinese patent application No. 201210258175.3 filed on July 24, 2012, entitled" Compound Preparation of Lercanidipine and Atorvastatin", the contents thereof are incorporated herein in its entirety by reference.

### FIELD OF THE INVENTION

The invention relates to the medical field, in particular relates to a compound preparation of lercanidipine and atorvastatin.

### BACKGROUND OF THE INVENTION

Hypertension was the most common cardiovascular disease in the Chinese populations, and was the leading cause of stroke and an important risk factor of coronary heart disease. In November, 2009, the American Society of Hypertension (ASH) updated the definition of hypertension as "a complex and progressing cardiovascular (CV) syndrome caused by a variety of disease causes including interactions therebetween". It is found in studies that hypertension is a kind of abnormal metabolism syndrome, wherein glucose and lipid metabolic disorders accompanied by hypertension are most common. Hyperlipidemia correlates closely with the morbidity and mortality of coronary heart disease and other atherosclerotic diseases, and is a risk factor of stroke, coronary heart disease, myocardial infarction and sudden death, and also an important risk factor of developing hypertension, abnormal glucose tolerance and diabetes. According to the epidemiological statistics data, the morbidity of hyperlipidemia in China is more than 10% at present, there are more than 100 millions of people throughout the country requiring lipid-regulating therapy, and in Europe and the United States, the dyslipidemia and cholesterol levels are higher than those of the Chinese populations. Numerous of studies have confirmed that there is a positive correlation between blood cholesterol level and blood pressure. In a study of 1032 patients with hypertension, 147 patients therein suffered from simple hypertension, accounting for only 14.3%; and 885 patients suffered from hypertension accompanied by various disorders, accounting for 85.7%, in which 15.0% was hypertension accompanied by dyslipidemia. In the populations above 35 years old, the patients having hypertension accompanied by dyslipidemia were up to 37 millions (30%~50% thereof accompanied by hyperlipidemia). Under the same blood pressure level, there was a positive correlation between blood cholesterol level and blood pressure.

In a study of Tromso, 8081 males aged 20-54 and 7663 females aged 20-49 were analyzed and found for both males and females, both levels of total cholesterol and non-HDL cholesterol were significantly increased as the systolic and diastolic blood pressure increased. The cardiovascular risk in patients with hypertension accompanied by dyslipidemia was not a simple sum thereof, but showing a situation of "1 + 1> 2". Now, the danger of hyperlipidemia accompanied by hypertension has been fully aware of, and thus positive antihypertensive treatment should be combined with lipid-lowering treatment.

Lercanidipine, developed by Recordati, an Italian company, is a third-generation dihydropyridine calcium channel antagonist. Lercanidipine hydrochloride had the similar mechanism of action as lercanidipine, i.e., reversibly blocking the Ca²⁺ influx in the L-type calcium channels on the cell membrane of the vascular smooth muscle and expanding the peripheral vessel so as to lower the blood pressure. Lercanidipine hydrochloride has a high lipophilicity, thus the onset time is delayed and the duration of the action is longer. In vivo and in vitro tests have shown that the negative inotropic effect caused by selective vasodilation effect of lercanidipine hydrochloride is weaker than nifedipine, nitrendipine and felodipine; while the selectivity on the vessel is stronger than amlodipine, felodipine, nitrendipine and lacidipine. Furthermore, lercanidipine hydrochloride also had an anti-atherosclerosis effect and a protective effect on end organs. Lercanidipine hydrochloride, in a therapeutic dosage, would not interfere with the normal cardiac excitability and conductivity in patients with hypertension. Animal experiments indicated that the product had a protective effect on kidney, and the mechanism thereof may be independent of hemodynamics. As compared with the same kind of drugs, lercanidipine hydrochloride had a stronger vascular selectivity, and the unique lipophilicity thereof contributed to a slower and longlasting antihypertensive effect, which is well accepted by the patients. The product has a high safety with no cardiac effects, does not affect the heart rate, and at the same time has a good anti-atherosclerotic effect, which is especially suitable for the patients with atherosclerosis accompanied by hypertension.

Atorvastatin is an inhibitor of 3-hydroxy-3-methylglutaryl coenzyme A (HMG-CoA) reductase, the main function of which is to reduce the level of plasma low-density lipoprotein cholesterol (LDL-C), which is a first-choice novel drug for lowering blood lipid and is currently used in clinical treatment of hypercholesterolemia (HC) and mixed hyperlipidemia characterized by increased total cholesterol (TC) and LDL-C. The substance *per se* has no activity, after being taken orally and absorbed, the hydrolyzates thereof competitively inhibit hydroxymethyl glutaryl coenzyme A reductase *in vivo,* a rate-limiting enzyme in the process of cholesterol synthesis, and reduce the synthesis of cholesterol and increases the synthesis of low density lipoprotein receptor. The main action site is liver, resulting in a lowered blood cholesterol and LDL cholesterol level, moderately lowered serum triglyceride level and elevated blood high-density lipoprotein level, and thereby functions in preventing and treating atherosclerosis and coronary heart disease.

Atorvastatin calcium tablet is the most widely used cholesterol-lowering drug worldwide. Atorvastatin can lower blood lipid level in a more effective way than other statins drugs. Comparing with the same dose of simvastatin, pravastatin and lovastatin, the lipid-lowering effect of atorvastatin calcium is improved by 38%, 46% and 51%, respectively. Atorvastatin calcium is a new synthetic statin drug currently most effective in lowering the total cholesterol. For patients with coronary heart disease, atorvastatin calcium tablet reduces the fatted cholesterol, increases the crystallized cholesterol, and enhances the mechanical strength of the plaque and makes it stabilized so as not easy to break. At the same time, atorvastatin calcium could also inhibit the proliferation of smooth muscle cell and the migration thereof to subintima, reducing the risk of plaque rupture and thus decreasing the acute coronary events and achieving a high lipid-lowering rate.

The combined administration of lercanidipine and atorvastatin for the treatment of hypertension has not been reported.

### SUMMARY OF THE INVENTION

The technical problem to be solved by the present invention is to provide a compound preparation of lercanidipine and atorvastatin calcium, comprising in one dosage unit 5-20 mg of lercanidipine hydrochloride and 10-80 mg of atorvastatin calcium. The compound preparation of the present invention can effectively control the blood pressure and lipid levels in patients with hypertension accompanied by hyperlipidemia, is convenient to be used and has a good compliance in animals.

Clinical studies and animal experiments have shown that atorvastatin has the effects of adjusting blood pressure and protecting heart. The higher the blood pressure is, the more remarkable the antihypertensive effect is, and its antihypertensive effect is independent of the lipid-lowering effect. Statins drugs are not antihypertensive drugs, and they mainly focus on the risk of cardiovascular disease and increase the antihypertensive effect of antihypertensive drugs. For the patients with hypertension, the treatment with statins drugs together with positive antihypertensive treatment can prevent the occurrence of cardiovascular event.

Preferably, the mass ratio of lercanidipine hydrochloride to atorvastatin calcium is 1:1-8.

More preferably, the mass ratio of lercanidipine hydrochloride to atorvastatin calcium is 1:2-4.

Preferably, the compound preparation comprises 1-5% of lercanidipine hydrochloride, 2-20% of atorvastatin calcium, and 0.01-20% of calcium carbonate, 10-40% of microcrystalline cellulose, 10-20% of sodium carboxymethyl cellulose, 2-8% of hydroxypropyl cellulose, 0.1-10% of polysorbate 80, 0.5-2.5% of magnesium stearate, 5-15% of pre-gelatinized starch and 1-10% of colloidal silicon dioxide as excipients.

Preferably, an Opadry sugar coating is used in an amount of 2-4% of the compound preparation.

The present invention also provides a method for preparing the compound preparation, comprising the following steps:
step 1: the active pharmaceutical ingredient lercanidipine hydrochloride and the active pharmaceutical ingredient atorvastatin calcium are weighed and sieved, mixed homogeneously with calcium carbonate, microcrystalline cellulose, sodium carboxymethyl cellulose, hydroxypropyl cellulose, polysorbate 80, magnesium stearate, pre-gelatinized starch and colloidal silicon dioxide, compressed by vacuum, milled and sieved to form granules, and compressed into tablets with the hardness of the tablet core controlled;
step 2: Opadry is added to ethanol under stirring and stirred until dispersion, then purified water is added and stirred to formulate a separate coating solution, which is then used to coat the tablet cores obtained in step 1 to form a thin film coating;
step 3: Opadry is added to ethanol under stirring, purified water is added and stirred to formulate a separate coating solution, which is then used to coat the resulting tablet cores to form a thin film coated tablet;
step 4: Opadry is added to ethanol and stirred homogeneously to formulate an enteric coating solution, which is then used to coat the thin film coated tablets to form an enteric coating.

Preferably, the hardness of the tablet core controlled in step 1 is 2-10 kg.

Preferably, the concentration of ethanol in steps 2 and 3 is 75-100%.

Preferably, the duration for adding and stirring the purified water in step 2 and for stirring in step 3 are 20-100 min.

Preferably, the Opadry in step 2 is Y-1-7000 and the Opadry in step 3 is OY-P, type 91S.

Animal experiments of the present invention show that, the combination administration of lercanidipine/atorvastatin significantly reduces the blood pressure and lipid levels of the rats with hypertension accompanied by dyslipidemia simultaneously, and each effect is slightly higher than that of the corresponding single component, indicating a synergistic effect between the two components.

Clinical trial results show that the combination administration of lercanidipine and atorvastatin has significant activities of both antihypertensive effect and lipid-lowering effect. As compared with the pre-administration, the difference is statistically significant (P<0.05), and the activity is slightly greater than the corresponding activity of the single drug. The single-component group has gastrointestinal symptoms, dizziness and other adverse reactions; while the combination administration group does not appear significant adverse reactions.

The above-mentioned results suggest that, the lercanidipine and atorvastatin compound preparation plays significant antihypertensive and lipid-lowering effects simultaneously, having a higher effectiveness with reduced pharmaceutical dosage, compared with combined administration of amlodipine besylate tablet and simvastatin tablet, and the incidence of adverse reactions is reduced, and a good tolerance and compliance is achieved in patients.

Formulating lercanidipine and atorvastatin into a compound preparation can have a synergistic effect in increasing the antihypertensive and lipid-lowering efficacy; while reducing adverse reactions induced by the drugs and increasing the tolerance and compliance in the patients. The compound preparation is convenient for administration for the patient and has a good prospect of clinical application.

### DETAILED DESCRIPTION OF THE INVENTION

The present invention discloses a lercanidipine and atorvastatin compound preparation, which can be achieved by those skilled in the art by virtue of the present description by appropriately improving the process parameters. It should be particularly noted that all similar substitutions and modifications are obvious for the skilled artisan, and are deemed to be within the scope of the present invention. The products and applications of the present invention have been described through preferred examples. Without departing from the content, the spirit and scope of the present invention, changes, appropriate modifications and combinations to the methods and applications described herein can be apparently made by the skilled artisan to realize and apply the techniques of this invention.

The present invention is further described in detail by combining the specific embodiments as follows in order that the technical solution of the present invention can be better understood by those skilled in the art.

### Example 1: Formula of the compound preparation of the present invention

The compound preparation comprises lercanidipine hydrochloride and atorvastatin calcium as the active pharmaceutical ingredients, and the excipients comprise calcium carbonate, sodium carboxymethyl cellulose, microcrystalline cellulose, pre-gelatinized starch, polysorbate 80, hydroxypropyl cellulose, purified water, colloidal silicon dioxide, magnesium stearate and Opadry sugar coating.

**Table1. Formula**

| Component | weight ratio(%) |
|---|---|
| Active pharmaceutical ingredients | |
| lercanidipine hydrochloride | 1-5 |
| atorvastatin calcium | 2-20 |

| Excipients | |
|---|---|
| calcium carbonate | 0.01~20 |
| microcrystalline cellulose | 10~40 |
| sodium carboxymethyl cellulose | 10~20 |
| hydroxypropyl cellulose | 2~8 |
| polysorbate 80 | 0.1~10 |
| magnesium stearate | 0.5~2.5 |
| pre-gelatinized starch | 5~15 |
| colloidal silicon dioxide | 1~10 |
| water | q.s. |

| Coating | |
|---|---|
| Opadry sugar coating | 2~4 |

### Example 2: Preparation of the compound preparation of the present invention

(1) The active pharmaceutical ingredient lercanidipine hydrochloride and the active pharmaceutical ingredient atorvastatin calcium were weighed and sieved for use.
(2) Calcium carbonate, microcrystalline cellulose, sodium carboxymethyl cellulose, hydroxypropyl cellulose, polysorbate 80, magnesium stearate, pre-gelatinized starch and colloidal silicon dioxide were weighed and mixed homogeneously with the active pharmaceutical ingredients; the excipients comprised 0.01-20% of calcium carbonate, 10-40% of microcrystalline cellulose, 10-20% of sodium carboxymethyl cellulose, 2-8% of hydroxypropyl cellulose, 0.1-10% of polysorbate 80, 0.5-2.5% of magnesium stearate, 5-15% of pre-gelatinized starch and 1-10% of colloidal silicon dioxide.
(3) The mixture was compressed by vacuum, milled and sieved to form granules, and compressed into tablets with the hardness of the tablet core controlled as 2-10 kg.
(4) Coated with thin-film coating: Opadry (Y-1-7000) was added to ethanol under stirring until dispersion, then purified water was added and stirred for 20-100 min to formulate a separate coating solution (the concentration of ethanol was 75-100%), which was used to coat the tablet cores to form a thin-film coated tablet.
(5) Coated with enteric coating: Opadry (OY-P, type 91S) in a prescribed amount was added to ethanol under stirring, and further stirred for 20-100 min, and the enteric coating solution was obtained (the concentration of ethanol was 75-100%), which was used to coat the thin-film coated tablets to form an enteric coating. Each tablet of the compound preparation prepared contained 5-10 mg of lercanidipine hydrochloride and 10-80 mg of atorvastatin calcium.

### Example 3: Quality Study Method

The description, identification, detection (related substances, loss on drying, chloride, heavy metals, dissolution rate and content uniformity) and content determination and so on were included. The determination of the contents of lercanidipine hydrochloride was as follows:
Content determining: HPLC method, the stationary phase and mobile phase used were listed in the following table.

**Table 2. HPLC conditions for determining the contents of lercanidipine hydrochloride in the compound preparation**

| Mobile phase | A: buffer |
|---|---|
| | B: acetonitrile |
| Column | C18, Waters 250 mm × 4.6 mm; 5µm |
| Flow rate | 1.0 ml/min |
| Detection wavelength | 210 nm |
| Column temperature | 50 °C |
| Loading amount | 20 µl |
| Running time | 120 min |
| Diluent | buffer: acetonitrile (50:50) |

Preparation of A buffer: 1.36 g of potassium dihydrogen phosphate was dissolved in 1

L of water, and 1 g of octane sulphonic acid sodium salt was added after the potassium dihydrogen phosphate was dissolved. The mixture was treated with ultrasonic processing and the pH was adjusted to 2.5 ± 0.05 using 10% phosphoric acid (v/v).

### Example 4: Antihypertensive and lipid-lowering experiments in rats with spontaneously hypertension accompanied by dyslipidemia

5-month-old rats with spontaneous hypertension (adult MHS rats), half males and half females, were fed with high fat diet every day (1.0% of cholesterol, 0.2% of propylthiouracil, 0.5% of sodium cholate, 5.0% of lard, 1.0% of methionine and 92.3% of basal diet) for four weeks; after four weeks, the basal systolic pressure and lipid levels in rats weighing 200-240 g were determined. Rats with hypertension accompanied with dyslipidemia were evenly divided into 4 groups with 12 rates in each group. The four groups were respectively as follows: solvent control group (A), lercanidipine group (lercanidipine hydrochloride, 0.4 mg/kg) (B), atorvastatin group (atorvastatin calcium, 0.8 mg/kg) (C), and lercanidipine/atorvastatin compound group (lercanidipine hydrochloride + atorvastatin calcium, 0.4mg/kg + 0.8 mg/kg) (D). Each group of drugs was formulated with physiological saline, and were administered to rats by gavage once a day for 4 consecutive weeks. Rats were measured 2 hours after administration at the end of each week for the systolic pressure and total serum cholesterol levels and so on.

Changes in systolic pressure in each group were shown in Table 3, the systolic pressure in groups B and D was changed significantly (p <0.01), and was decreased to normal blood pressure levels, and the degree of decrease in D group was more than that in group B. There was no significant change in the systolic pressure in group A. Changes in total cholesterol in each group were shown in Table 4, wherein there were decrease in groups B, C and D, and decreases in groups C and D were significant (p <0.05). The total cholesterol in group B was decreased (p> 0.05), whereas no significant change happened in group A.

**Table 3. Changes in systolic pressure in each group (Mean±SD) (mmHg)**

| Group | 0 week | 1 week | 2 weeks | 3 weeks | 4 weeks |
|---|---|---|---|---|---|
| A | 165±4.3 | 168±2.5 | 170±3.3 | 169±5.3 | 172±6.0 |
| B | 162±4.1 | 158±3.7 | 149±5.1 | 138±3.4 | 135±2.5 |
| C | 160±5.3 | 166±2.9 | 161±5.1 | 159±4.2 | 158±3.7 |
| D | 168±1.9 | 155±3.6 | 144±3.1 | 131±1.5 | 129±2.5 |

**Table 4. Changes in total cholesterol in each group (Mean±SD)(mmol/L)**

| Group | 0 week | 1 week | 2 weeks | 3 weeks | 4 weeks |
|---|---|---|---|---|---|
| A | 8.58±1.20 | 8.49±2.05 | 8.37±1.87 | 8.65±2.18 | 8.56±1.95 |
| B | 8.49±3.01 | 7.95±2.48 | 7.55±1.94 | 7.28±1.76 | 7.01±1.77 |
| C | 8.75±2.65 | 7.51±1.79 | 6.67±2.03 | 6.15±1.47 | 5.72±1.48 |
| D | 8.69±1.01 | 7.38±2.12 | 6.55±2.05 | 5.83±1.60 | 5.45±1.78 |

It can be seen that, combination administration of lercanidipine and atorvastatin in normal dosage can simultaneously and effectively control the blood pressure and blood lipid of the rats with hypertension accompanied by hyperlipidemia, and is convenient for administration and has a good compliance in animals.

### Example 5: Antihypertensive and lipid-lowering experiments in experimental rats with hypertension accompanied by dyslipidemia

SD rats, weighing 150-180 g, were anaesthetized by intraperitoneal injection with chloral hydrate (320mg/kg). The abdominal cavity was opened, and the left renal artery was separated and was restricted with 0.2 mm silver clip. At 8-10 weeks following the procedure, rats with systolic pressure ≥ 140 mmHg were recognized as hypertension rats. Rats with hypertension were administered with vitamin D3 by gavage in a total dosage of 600,000 U/kg in three days, and then were fed with high-fat diet every day (cholesterol 1.0%, propylthiouracil 0.2%, sodium cholate 0.5%, lard 5.0%, methionine 1.0%, basal diet 92.3%). Rats in normal control group were fed with normal diet. After four weeks, systolic pressure was measured in rats, serum total cholesterol (TC) and triglyceride (TG) levels were determined by taking blood from tail vein.

140 rats with hypertension accompanied by dyslipidemia were evenly divided into 7 groups with 20 rats in each group according to blood pressure and lipid levels, which were respectively solvent control group, lercanidipine group (lercanidipine hydrochloride, 0.5 mg/kg), atorvastatin group (atorvastatin calcium, 1.0 mg/kg), lercanidipine/ atorvastatin compound group 1 (lercanidipine hydrochloride + atorvastatin calcium, 0.5 mg/kg + 0.5 mg/kg), lercanidipine/ atorvastatin compound group 2 (lercanidipine hydrochloride + atorvastatin calcium, 0.5 mg/kg + 1.0 mg/kg), lercanidipine/ atorvastatin compound group 3 (lercanidipine hydrochloride + atorvastatin calcium, 0.5 mg/kg + 2.0 mg/kg), and positive control group (amlodipine besylate + atorvastatin calcium, 0.5 mg/kg + 1.0 mg/kg).

Another 20 normal rats served as normal control group. Rats with hypertension were continued to be fed with the high-fat diet, and rats in normal group were fed with normal diet. Each group of drugs was formulated with physiological saline, and for the normal control group and solvent control group rats, same volume of physiological saline were administered. Rats were administered by gavage once a day for 20 consecutive weeks. Rats were weighed once a week, and the dosage was adjusted based on the weight. Blood pressures in different time points before and after administration were measured respectively to calculate the magnitude of the decrease in blood pressure (systolic pressure before administration - systolic pressure after administration). Rats were sampled 20 weeks later, and the serum total cholesterol (TC) and triglyceride (TG) levels were determined. The corresponding parameters for each group were represented with the mean ± standard deviation (Mean ± SD), and t-test was used to compare different groups.

As shown in Table 5, at the end of week 20, compared with the rats in normal group, rats in the solvent control group had significantly increased systolic pressure; as compared with those of the solvent control group, systolic pressure of the atorvastatin group (1.0 mg/kg) was improved, all the systolic pressures of the lercanidipine group (0.5 mg/kg), lercanidipine/atorvastatin compound groups (lercanidipine hydrochloride + atorvastatin calcium, 0.5 mg/kg + 0.5/1.0/2.0 mg/kg), positive control group (amlodipine besylate + atorvastatin calcium, 0.5 mg/kg + 1.0 mg/kg) were significantly decreased (p<0.01) to the normal level, and as compared with the lercanidipine group (0.5 mg/kg), rats of the lercanidipine/atorvastatin compound groups (lercanidipine hydrochloride + atorvastatin calcium, 0.5 mg/kg + 0.5/1.0/2.0 mg/kg) had a slightly greater degree of decrease in systolic pressure, which yet did not have statistical significance. By the end of week 20, as compared with the rats of the normal group, TC and TG levels of the rats of the solvent control group increased significantly; and as compared with the solvent control group, TC and TG of the lercanidipine group (0.5 mg/kg) had been improved, TC and TG of the atorvastatin group (1.0 mg/kg), lercanidipine/atorvastatin compound groups (lercanidipine hydrochloride + atorvastatin calcium, 0.5 mg/kg + 0.5/1.0/2.0 mg/kg), positive control group (amlodipine besylate + atorvastatin calcium, 0.5 mg/kg + 1.0 mg/kg) were all significantly decreased (p<0.01), and as compared with the atorvastatin group (1.0mg/kg), rats of the lercanidipine/ atorvastatin compound group 2 (lercanidipine hydrochloride + atorvastatin calcium, 0.5 mg/kg + 1.0 mg/kg) had a greater degree of decrease in TC and TG. As compared with the positive control group (amlodipine besylate + atorvastatin calcium, 0.5 mg/kg + 1.0 mg/kg), the antihypertensive and lipid-lowering effects of lercanidipine/ atorvastatin compound group 2 (lercanidipine hydrochloride + atorvastatin calcium, 0.5 mg/kg + 1.0 mg/kg) were slightly higher (which yet did not have high statistical significance), and was likely to relate with stronger lipotropism of lercanidipine.

**Table 5. Effects of lercanidipine + atorvastatin on the blood pressure and the lipid level of the rats with hypertension accompanied by dyslipidemia (Mean±SD)**

| Group | Dosage mg/kg | Degree of antihypertension (mmHg) | TC (mmol/L) | TG (mmol/L) |
|---|---|---|---|---|
| Normal group | - | -3.8±2.2 | 1.44±0.56 | 0.82±0.35 |
| Solvent control group | - | -26.1±6.5** | 8.25±3.01** | 2.20±1.06** |
| Lercanidipine group | 0.5 | 35.8±9.65^{§§} | 8.27±2.96 | 2.43±1.21 |
| Atorvastatin group | 1.0 | 2.4±1.98 | 5.27±4.03^{§§} | 1.49±0.74^{§§} |
| Lercanidipine/ atorvastatin compound group (lercanidipine hydrochloride + atorvastatin calcium) | 0.5+0.5 | 36.3±8.36^{§§} | 5.83±3.45^{§§} | 1.66±1.17^{§§} |
| | 0.5+1.0 | 36.6±6.54^{§§} | 5.19±3.74^{§§} | 1.42±0.81^{§§} |
| | 0.5+2.0 | 37.0±9.12^{§§} | 4.98±2.78^{§§} | 1.40±0.46^{§§} |
| Positive control group (amlodipine besylate + atorvastatin calcium) | 0.5+1.0 | 35.7±8.52^{§§} | 5.73±2.56^{§§} | 1.58±0.71^{§§} |

As compared with the normal group, **p<0.01; as compared with the solvent control group, §p<0.05, §§p<0.01.

It can be seen that, combination administration of the lercanidipine/ atorvastatin (lercanidipine hydrochloride + atorvastatin calcium, 0.5mg/kg + 0.5/1.0/2.0 mg/kg) decreased the blood pressure and lipid levels of the rats with hypertension accompanied by dyslipidemia simultaneously and significantly, and each effect was higher than that of the corresponding single component. The two components had certain synergistic effect.

### Example 6: Therapeutic effect of the administration of lercanidipine and atorvastatin alone and the combination administration thereof on patients with hypertension accompanied by hyperlipidemia

A double-blind, parallel, randomized study on the effect of the combination administration of lercanidipine and atorvastatin on simultaneously controlling hypertension and hyperlipidemia was conducted in patients with moderate hypertension and hyperlipidemia.

Inclusion criterion for the subjects: the subjects were adult males or females aged between 18 and 80 years old simultaneously suffering from moderate hypertension and hyperlipidemia.

The presence of hyperlipidemia can be confirmed by evaluating the low density lipoprotein (LDL) level in the subject associated with certain positive risk factors. If a subject had no coronary heart disease (CHD) and had less than two positive risk factors, when the LDL of the subject was greater than or equal to 190 mg/dl, the subject was considered as having hyperlipidemia in need of drug therapy; if a subject had no CHD and had two or more risk factors, when the LDL of the subject was greater than or equal to 160 mg/dl, the subject was considered to have hyperlipidemia in need of drug therapy; and if a subject suffered from CHD, when the LDL of the subject was greater than or equal to 130 mg/dl, the subject was considered to have hyperlipidemia. The positive risk factors include:(1) male over 45 years old, (2) female over 55 years old, wherein the female did not undergo hormone replacement therapy (HRT), (3) family history of early onset of cardiovascular disease, (4) the subject is a regular smoker, (5) the subject has diabetes, (6) HDL less than 45mg/dl, and (7) the subject suffers from hypertension. HDL greater than 60mg/dl was considered to be a negative risk factor, which can counteract one of the above positive risk factors.

When resting diastolic blood pressure is between 105 and 114 mmHg, the subject is believed to suffer from the moderate hypertension. All the blood pressures are the average of three measurements with a 5-minute interval.

The subjects who met the inclusion criterion stopped taking the current anti-hypertension drugs and lipid-lowering drugs and allowed almost all of the drugs eliminated from the body of the subjects. The newly diagnosed subjects generally did not receive a treatment before the test started. After 4 weeks, the basal measurement was determined for the blood pressure and fasting lipid level of the subjects, the total cholesterol, LDL-cholesterol, HDL-cholesterol, triglycerides, very low density lipoprotein (VLDL) and the like were determined in examination of the lipids.

Thereafter, the subjects were randomly divided into the following three groups:
(1)lercanidipine group (lercanidipine hydrochloride tables, Recordati S.P.A., 10mg/tablet);
(2) atorvastatin group ( atorvastatin calcium tables, Pfizer Ireland Pharmaceuticals, 40mg/table);
(3)lercanidipine + atorvastatin group (compounding tablets of lercanidipine/ atorvastatin 10mg/40 mg prepared according to the preparation method described above).

Each group of 50 subjects took one tablet of the corresponding drugs once every day at half an hour before dinner for 8 weeks. During the test, the subjects were required to normal diet, without taking any other medications any more. After eight weeks, the subjects returned the testing centers, and were measured the blood pressure and lipid level again. Adverse reactions suffered by the subjects during the period of taking drugs were recorded, and prior to and after the test, complete detections including electrocardiogram, routine examinations on blood and urine, liver and kidney functions, blood glucose and blood electrolytes were conducted on the patients. The mean and standard deviation of the blood pressure and lipid parameters of each group were calculated (see Table 6), and t test was used to determine the difference level between pre-administration and post-administration.

**Table 6. Effects of lercanidipine and atorvastatin alone and combination administrations on the systolic pressure, lipid level (TC, LDL-C and TG) in patients with hypertension accompanied with hyperlipidemia (Mean±SD)**

| Group | systolic pressure (mmHg) | | TC (mmol/L) | | LDL-C (mmol/L) | | TG (mmol/L) | |
|---|---|---|---|---|---|---|---|---|
| | 0 week | 8 weeks | 0 week | 8 weeks | 0 week | 8 weeks | 0 week | 8 weeks |
| 1 | 159±5 | 133±5 ** | 7.01±0.45 | 7.12±0.78 | 3.55±0.63 | 7.01±0.45 | 2.66±0.39 | 2.60±0.55 |
| 2 | 160±7 | 157±6 | 7.14±0.36 | 4.93±0.64 ** | 3.49±0.75 | 2.97±0.58 ** | 2.70±0.48 | 1.72±0.48 ** |
| 3 | 161±7 | 130±8 ** | 7.06±0.71 | 4.85±0.48 ** | 3.56±0.83 | 2.95±0.74 ** | 2.63±0.49 | 1.68±0.73 ** |

| | | | | | | | | |
|---|---|---|---|---|---|---|---|---|
| Compared with the basal value in week 0, **p<0.05. | | | | | | | | |

The test results showed that the combination administration of the lercanidipine and atorvastatin simultaneously played antihypertensive and lipid-lowering activities, and the difference was statistically significant compared with the pre-administration (P <0.05), and the activity was slightly stronger compared with the corresponding activity of single drug. The group with alone administration of lercanidipine had 1 case of gastrointestinal symptoms, 1 case of dizziness; the group with alone administration of atorvastatin had 1 case of dry mouth and 1 case of abdominal pain, both were mild and did not affect the continued administration. The combination administration group did not show significantly adverse reactions. No significant difference was shown after eight weeks of treatment with respect to the change of laboratory check results and electrocardiogram between the three groups of patients.

### Example 7: Comparison between the effect of lercanidipine atorvastatin compound preparation and the effect of combined administration

Combined administration: administration of two drugs, amlodipine besylate tablets (Zhejiang Weikang Pharmaceutical Co. Ltd., 5mg/tablet) and simvastatin tablets (Merck Sharp & Dohme (Australia) Pty Ltd., 20mg/tablet), in combination.

Effects of lercanidipine atorvastatin compound preparation and the above combined administration on patients with moderate hypertension accompanied by hyperlipidemia were evaluated.

Inclusion criterion for the subjects: the subjects were adult males or females aged between 18 and 80 years old simultaneously suffering from moderate hypertension and hyperlipidemia. The evaluation indicators were the same as previous example.

The subjects who met the inclusion criterion stopped taking the current anti-hypertension drugs and lipid-lowering drugs and allowed almost all of the drugs eliminated from the body of the subjects. The newly diagnosed subjects generally did not receive a treatment before the test started. After 4 weeks, the basal measurement was determined for the blood pressure and fasting lipid level of the subjects, and the total cholesterol, LDL-cholesterol, HDL-cholesterol, triglycerides, very low density lipoprotein (VLDL) and the like were determined by examination of the lipids. There were 98 patients in total who participated in the test. The subjects were randomly divided into the following two groups according to the blood pressure and lipid level (49 patients in each group):
(1)lercanidipine atorvastatin compound preparation group (compounding tablets of lercanidipine/ atorvastatin 10mg/20 mg prepared according to the preparation method described above);
(2)combined administration group of amlodipine besylate tablet and simvastatin tablet.

Each group of subjects took the corresponding drugs once every day at half an hour before dinner for 8 weeks. One tablet was taken each time for the compound preparation group, while one tablet of each drug was taken each time (2 tablets in total) for the combined administration group. During the test, the subjects were required to have normal diet, without taking any other medications any more. After eight weeks, the subjects returned the testing centers, and the blood pressure and lipid level were measured again. Adverse reactions suffered by the subjects during the administration period were recorded. The mean and standard deviation of the blood pressure and lipid parameters of each group were calculated (see Table 7), and t test was used to determine the differences between pre-administration and post-administration and between the two groups.

**Table 7. Effects of lercanidipine atorvastatin compound tablet and combined administration of amlodipine besylate tablet and simvastatin tablet on the systolic pressure and lipid level in patients with hypertension accompanied by hyperlipidemia (Mean±SD)**

| Group | systolic pressure (mmHg) | | TC (mmol/L) | | LDL-C (mmol/L) | | TG (mmol/L) | |
|---|---|---|---|---|---|---|---|---|
| | 0 week | 8 weeks | 0 week | 8 weeks | 0 week | 8 weeks | 0 week | 8 weeks |
| 1 | 157±5 | 135±5 ** | 6.92±0.64 | 5.02±0.39 ** | 3.50±0.73 | 2.88±0.39 ** | 2.58±0.79 | 1.75±0.45 ** |
| 2 | 155±7 | 138±6 ** | 7.01±0.45 | 5.16±0.71 ** | 3.47±0.48 | 2.93±0.43 ** | 2.63±0.41 | 1.77±0.68 ** |

| | | | | | | | | |
|---|---|---|---|---|---|---|---|---|
| Compared with the basal value at week 0, **p<0.05. | | | | | | | | |

Amlodipine, is used as a representative calcium channel blocker, particularly dihydropyridine calcium channel blocker. Its combination with simvastatin, a lipid-lowering statin drug, is a most widely prescribed formula. The test results showed that, both of lercanidipine atorvastatin compound tablet and combined administration of amlodipine besylate tablet and simvastatin tablet significantly reduced the blood pressure and lipid level in patients with moderate hypertension accompanied by hyperlipidemia. For the compound preparation group, only one case of mild ankle edema, no subjects quit the test due to the intolerance of adverse reactions; and for the combined administration group, one case of moderate edema, one case of mild headache, and one subject quit the test due to the adverse reactions of the drugs. Despite the difference in efficacy between the two groups was not statistically significant, but better antihypertensive and lipid-lowering effects were achieved in the lercanidipine atorvastatin compound preparation group and better tolerance and compliance in the patient were observed.

In conclusion, lercanidipine/atorvastatin compound preparation simultaneously plays significant antihypertensive and lipid-lowering effects, having higher effectiveness with less dosage, as compared with the combined administration of amlodipine besylate tablet and simvastatin tablet, and has reduced incidence of adverse reactions and a good tolerance and compliance in patients.

The above descriptions are only preferred embodiments of the present invention. It should be noted that those of ordinary skill in the art can further make numbers of improvements and modifications without departing from the principles of the invention, which should also be regarded as within the scope of protection of the invention.

## Claims

1. A compound preparation of lercanidipine and atorvastatin calcium, **characterized in that**, 5-20 mg of lercanidipine hydrochloride and 10-80 mg of atorvastatin calcium are comprised in per unit dosage form.

2. The compound preparation according to claim 1, **characterized in that**, the mass ratio of lercanidipine hydrochloride to atorvastatin calcium is 1:1-8.

3. The compound preparation according to claim 1, **characterized in that**, the mass ratio of lercanidipine hydrochloride to atorvastatin calcium is 1:2-4.

4. The compound preparation according to claim 1, **characterized by** comprising 1-5% of lercanidipine hydrochloride, 2-20% of atorvastatin calcium, and 0.01-20% of calcium carbonate, 10-40% of microcrystalline cellulose, 10-20% of sodium carboxymethyl cellulose, 2-8% of hydroxypropyl cellulose, 0.1-10% of polysorbate 80, 0.5-2.5% of magnesium stearate, 5-15% of pre-gelatinized starch and 1-10% of colloidal silicon dioxide as excipients.

5. The compound preparation according to claim 4, **characterized in that**, an Opadry sugar coating is used in an amount of 2-4% of the compound preparation.

6. A method for preparing the compound preparation according to claim 5, **characterized by** comprising the following steps:
step 1: the active pharmaceutical ingredient lercanidipine hydrochloride and the active pharmaceutical ingredient atorvastatin calcium are weighed and sieved, mixed homogeneously with calcium carbonate, microcrystalline cellulose, sodium carboxymethyl cellulose, hydroxypropyl cellulose, polysorbate 80, magnesium stearate, pre-gelatinized starch and colloidal silicon dioxide, compressed by vacuum, milled and sieved to form granules, and compressed into tablets with the hardness of the tablet core controlled;
step 2: Opadry is added to ethanol under stirring and stirred until dispersion, then purified water is added and stirred to formulate a separate coating solution, which is then used to coat the tablet cores obtained in step 1 to form a thin film coating;
step 3: Opadry is added to ethanol under stirring, purified water is added and stirred to formulate a separate coating solution, which is then used to coat the resulting tablet cores to form a thin film coated tablet;
step 4: Opadry is added to ethanol and stirred homogeneously to formulate an enteric coating solution, which is then used to coat the thin film coated tablets to form an enteric coating.

7. The preparation method according to claim 6, **characterized in that**, the hardness of the tablet core controlled in step 1 is 2-10 kg.

8. The preparation method according to claim 6, **characterized in that**, the concentration of ethanol in steps 2 and 3 is 75-100%.

9. The preparation method according to claim 6, **characterized in that**, the duration for adding and stirring the purified water in step 2 and for stirring in step 3 are 20-100 min.

10. The preparation method according to claim 6, **characterized in that**, the Opadry in step 2 is Y-1-7000 and the Opadry in step 3 is OY-P, type 91S.
